Europäisches Patentamt

(19) European Patent Office

Office européen des brevets

(11) Publication number : **0 437 408 A2**

## EUROPEAN PATENT APPLICATION

(21) Application number : **91400065.8**

(22) Date of filing : **11.01.91**

(51) Int. Cl.⁵ : **G01N 33/48, G02B 21/34, B01L 3/00**

(30) Priority : **12.01.90 IL 93043**

(43) Date of publication of application :
**17.07.91 Bulletin 91/29**

(84) Designated Contracting States :
**AT BE CH DE DK ES FR GB GR IT LI LU NL SE**

(71) Applicant : **UNITED MEDICAL SYSTEMS ISRAEL LTD.**
**P.O. Box 9697**
**Haifa 31096 (IL)**

(72) Inventor : **Lightman, Abraham, Dr.**
**57 Palmach Street**
**Haifa (IL)**

(74) Representative : **Gutmann, Ernest et al**
**S.C. Ernest Gutmann - Yves Plasseraud 67,**
**boulevard Haussmann**
**F-75008 Paris (FR)**

(54) A disposable device for determining the quality of sperm cells.

(57)    A disposable single use device for an objective and accurate determination of sperm cells quality at a controlled constant temperature is described. The device comprises a chamber possessing a planar lower rectangular optical translucent solid surface possessing at least one grid of 1 square mm, on which there is an upper rectangular flat optical translucent solid surface at a precise distance selected in the range of between 10 to 100 microns. The device is located into a temperature controlled holding unit,placed under the microscope lens through which the evaluation and counting of the sperm cells are determined.The device is very simple and provides reliable counting of sperm cells, compared with other known expensive chambers used for this purpose.

EP 0 437 408 A2

# A DISPOSABLE DEVICE FORD DETERMINING THE QUALITY OF SPERM CELLS

The present invention relates to a device for determining the quality of sperm cells. More particularly the invention relates to a temperature controlled disposable single use device for determining the concentration, motility, vitality and morphology of sperm cells.

## BACKGROUND OF THE INVENTION

Male factors are estimated to be responsible in some 30-40% infertility of cases of married couples. As known, among the parameters to be considered for estimating the potential fertility of an ejaculate,the concentration and total number of normally moving sperm cells are of high importance. It is also known that sperm cells leaving the testes are substantially immotile, however during transit through the epydidymis they develop the capacity for progressive motility.

The most common method for evaluation of sperm concentration and motility involves a direct observation through a microscope.The method involves calculating the sperm cell concentration based on a single or more readings using the haemacytometer so called Neubauer chamber. The main disadvantages of this method are:
  – its inaccuracy due to the rapid movement of the cells at high magnification ;
  – the tedious operation involved, and
  – the effects of various room temperatures on the motility quality and its subjective approach ; mean differences of more than 20% in sperm concentration are to be expected between duplicate determinations even by the same person.

Some improvements in accuracy were later on suggested by filling in the haemocytometers counters by means of interferometry.

According to U.S. Patent No. 3,829,216 a method and apparatus are suggested for limiting the movements of sperm cells. The method involves the addition of a primer solution to the sperm solution in order to stick the heads of sperm cells. The two solutions are introduced into a shallow chamber, having a depth in the order of 15 to 50 microns wherein the cells adhere to the chamber walls and they can be readily counted by a conventional microscope. This method does not give any indication regarding the quality of the movement of the sperm cells. Also, since the depth of the chamber is not exact and constant, there is a danger that the counting will relate to several layers of sperm cells and thus the entire method would not provide a reliable result.Another disadvantage of the method is the fact that it is time consuming since the counting can start only after all the sperm cells become sticked (about 15 minutes at room temperature). A further disadvantage of the device is connected with the relatively high costs of the chamber and therefore it can not be disposable i.e. for single-use. Another device suggested for counting the sperm cells is the so called Makler chamber (Fertility and Sterility, Vol. 30, No. 3, September 1978). The chamber consists of two discs of a high quality optical flat glass. On the periphery of the lower disc, there are three pins raised exactly 10 microns above the plane, being equidistant located. As mentioned in this paper, the main disadvantage of the evaluation the sperm cells using a microscope is the thickness of the examined drop which can range from few micrometers to several tenths of them. According to this device using the particular disc,an exact chamber having 10 microns depth is obtained. This chamber is used together with a special still camera microphotography technique wherein a multiple exposure is achieved by a stroboscope. It is claimed that high accuracy may be obtained. The main disadvantage of this device, seems to be connected with the uneven distribution of the sperm cells within the chamber, especially in view of the viscid sample characteristic. Since the examination of the sperm cells is performed at room temperature, which can be very different from one laboratory to another, it is very difficult to compare motility quality parameters of semen between different laboratories, especially in view of the effect of temperature on the sperm motility. Another disadvantage is connected with the relatively expensive cost of the device fact which precludes its disposal after every count use. In these days, with the sharp rise and spread of the AIDS disease and the presence of the Human T-cell Lymphocyte virus (HTLV) in the semen of infected patients, there is a great need for a disposable sperm counting chamber to minimize the risk of infection. This risk as well as the risk of other infections diseases (e.g. hepatitis B) exist in the current chambers now in use, during the washing and the cleaning of the chamber after each examination.

It is an object of the present invention to provide a simple device which enables an easy and very accurate determination of sperm cells concentration and motility. It is another object of the present invention to provide a disposable inexpensive device which enables an easy and very accurate determination of sperm cells concentration and motility. It is yet a further object of the present invention to provide a device which would provide reproducible counting and evaluation of sperm cells concentration and motility at a controlled constant temperature.

## BRIEF DESCRIPTION OF THE INVENTION.

The invention relates to a single use, disposable device for an objective determination of sperm cells quality, which comprises a chamber possessing a planar lower rectangular optical translucent solid surface on which there is at least one grid which possesses an area of 1 square mm, means being provided around said grid to maintain a precise distance selected in the range of between 10-100 microns from said lower solid surface to an upper rectangular flat optical translucent solid surface, the two solid surfaces being strongly attached. The simplest method for counting the sperm is by a common microscope. Of course any type of microscope with various condensors and dark field can be successfully utilized, as well as an automated semen analysis systems. The disposable counting device is inserted into a temperature controlled holding unit which is placed under the microscope lens enabling to observe and count the movements of the sperm cells in the counting device. The temperature controlled holder is connected to the temperature control electronic instrument which regulates and keeps the holding units' temperature at the desired temperature, generally at 35°C-37°C.

## BERIEF DESCRIPTION OF THE DRAWINGS

FIG. 1 is a top plan view of the lower surface of the chamber with a most preferred embodiment.

FIG. 2 is a top plan view of the cover slide.

FIG. 3 is a top plan view of the grid.

FIG. 4 is a top plan view of the temperature controlled holding unit.

## DESCRIPTION OF THE PREFERRED EMBODIMENTS.

The chamber according to the present invention, being simple and inexpensive, is disposable after one time use. In view of the fact that the percentage of moving sperm cells and their mean velocity is influenced markedly by the temperature, it is suggested that the counting of the cells should be determined at a constant temperature in a biological range of 35 -37°C. According to the present invention this can be easily carried out by introducing the chamber into a temperature control unit kept at this temperature range. This will also assure that all the evaluations and countings will be reproducible and independently on the laboratory or technician conducting these determinations.

Various embodiments may be conceived for obtaining an exact distance between the two solid surfaces. According to a most preferred embodiment, the lower surface possesses designed paint bands, having a thickness of 0.01 mm printed around the grid with a precise height in the selected range of between 10 to 100 microns. This may be considered a simple and most inexpensive manner to get the required distance between the two flat surfaces. Of course the paint should be of a stable and resistant quality to the environment and inert towards the sperm cells. A number of various paints can be suggested such as those of the epoxy type.

According to another embodiment, it is possible to obtain said lower flat surface which already possesses a continuous round protrusion, at the selected height around the grid on which the upper flat surface will be attached and continuing the operation as in the previous case.

One may also conceive to obtain the chamber with three components, wherein there is a middle slide with a desired thickness, possessing a round hole which corresponds to one which surrounds the grid.

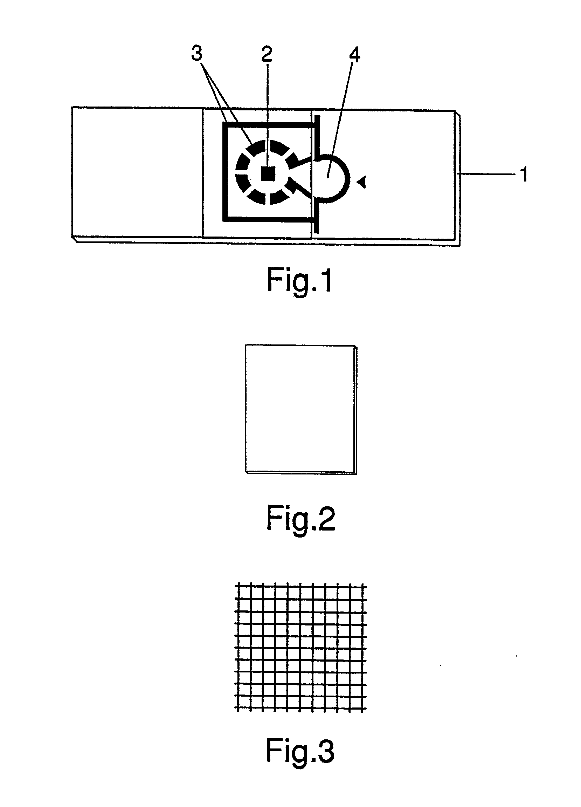

According to a most preferred embodiment, as illustrated in Figures 1-3, to be useful in case of normal sperm cells, the chamber consists of two microscopic slides made, from plastic or glass, generally the lower one with a size of 75x25 mm and the cover slide of 20 x 25 mm. The lower slide (1) is marked by a high resolution laser beam to obtain an exact 10x10 squares grid (2). The grid lines have a width of up to 7 microns. By obtaining this grid with the aid of the laser beam, it will be possible to obtain an easy and very accurate counting of the sperm cells under the microscope. Around the grid, there are specially designed paint bands (3) printed around, being raised precisely in the range of 9 to 11 microns above the slide. In this manner by covering this lower slide with an upper slide and strongly attaching them, a chamber of the corresponding thickness will be obtained.

The attachement of the slide with the cover slide is preferably done by gluing using a common glue such as cyanolit, although one may conceive to use any other method or other material for gluing. The gluing of the slides, ensures that the thickness of the two slides will be always constant and only a monolayer sperm cell will be counted.

The lower slide possesses an opening (4) through which the sample of sperm cells is introduced for counting. Generally the sample volume needed for the counting is about 10 to 20 microliters and is introduced conveniently in the round surface by conventional means such as a micropipette.

According to another embodiment, it is possible to produce the lower slide with two sets of the grid and means to provide the desired distance from two upper slides, thus obtaining a two-chamber slide. In this case it will be possible to examine two semen samples or duplicates of the same patient.

The chamber as mentioned above can be made from any solid material which is optical translucent and inert towards the sperm cells. Examples of such materials from which it can be produced are glass, teflon, polycarbonate etc.,but glass seems to be the most convenient material.

The chamber according to the present invention is characterized by its very low costs and a most reliable counting compared with other known expensive chambers.

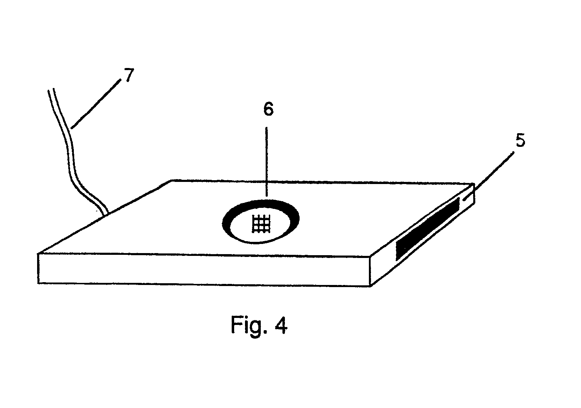

The temperature controlled holding unit, as illustrated in Figure 4, comprises a conductive plate, heated by electric elements,on which the counting device is placed. Generally said plate is made from a common metal, a most preferred one being aluminum. The units' body and walls are made of plastic or any other isolating material. A slot of the units' side (5) enables the insertion of the counting device so that the grid will be located at the center of the opening (6) through which the microscope observation is performed. The holding unit is connected via an electronic cable (7) to a temperature control instrument which can be adjusted to keep the holding unit temperature at a constant value, usually in the range of between 35°C-37°C.

In the following Table 1,there are summarized the results obtained with the chamber according to the present invention and compared with other well known chambers Makler and Neubauer.

TABLE 1: $\underline{\text{Sperm cells counting of 5 samples using three types of chambers (x } 10^6 \text{ cells/ml)}}$.

| Sample No. | Makler | Neubauer | The present invention |
|---|---|---|---|
| 1 | 36 | 41 | 32 |
| 2 | 20 | 24 | 17 |
| 3 | 84 | 105 | 90 |
| 4 | 2.5 | 3.2 | 2 |
| 5 | 104 | 143 | 115 |

The above results clearly show that the chamber of the present invention provides reliable countings compared with other well known expensive chambers used for this purpose.

In addition to its low costs which affords its disposal after one time use, the chamber has a number of distinct advantages which contribute to a reliable counting of the sperm cells. Thus, the counting with the present chamber is carried out on the ejaculate itself in the absence of a second liquid and without any limitation in the motility of the cells such as mentioned in the above U.S.Patent No. 3,829,216.

The simple provision according bo the preferred embodiment of the present invention to obtain an exact thickness between the two slides, enables to ensure that the counting will be effected only on one single layer of the sperm cells.

The cells are counted on the squares carved in the grid on the surface containing 10x10 lines with an exact total surface of 1 square mm. Thus the following simple formula will give the exact counting :

$$C = N \times 10^6$$

wherein :

C = concentration of the sperm cells in the sample (number of cells per ml).

N = the number of cells counted in 10 squares.

Another important advantage of the chamber according to the present invention, is the rapid counting which can be done at a constant temperature. The counting is done with the slide in the temperature controlled unit. As already mentioned above, it is most desirable that such countings should be carried out at a constant temperature of 36°C generally suggested for such measurements.

A further advantage of the chamber, is the fact that in addition to a reliable counting of the sperm cells, it is possible to get an indication on the quality of the motility, the morphology and index of activity of said cells, parameters which are very important for defining the fertility of men semen.

Summing up, the device according to the present invention has a number of important advantages :

– It provides a simple and accurate counting of the sperm cells at a constant temperature, controlled by a holding unit.

– It is disposable, being quite inexpensive and can be easily manufactured.

– The device can be used by any laboratory technician and even by unskilled persons.

– The device can be successfully utilized in research laboratories to determine the influence of various compounds on the motility of sperm (e.g. influence of caffeine, kallikrein etc.)

– The results obtained by the device compare favourable with those obtained by using other very expensive chambers. Moreover, since the device also enables to determine several parameters in one single sample, the results are more reliable than those of other chambers where several samples have to be analysed for determining the various parameters.

Although in the above description the device is described for counting the human male sperm cells, it can also be useful for determining the fertility of animal, particularly for the animals with the sperm concentration and sizes of the same order of magnitude as for those of men, such as dogs, bull, stallion, rabbit etc. In those cases where the sperm concentration is especially high, one can dilute the semen with any physiological solution (e.g. culture media, saline) before applying the sample in the chamber for counting. This dilution will not interfere or harm the counting.

While the invention has been described in connection with specific embodiments thereof, it will be understood that it is capable of further modifications, and it is intended to cover any variations, uses or departures from the present disclosure as come within known or customary practice in the art to which the invention pertains and as may be applied to the essential features hereinbefore set forth and as fall within the scope of the invention as defined by the attached Claims.

## Claims

1. A disposable device for an objective determination of sperm cells qualify at a constant temperature which comprises a chamber possessing a planar lower rectangular optical translucent solid surface on which there is at least one grid which possesses an area of 1 square mm, means being provided around said grid to maintain a precise distance selected in the range of between 10-100 microns from said lower solid surface to an upper rectangular flat optical translucent solid surface, the two solid surfaces being strongly attached.

2. The disposable device according to Claim 1, wherein said lower surface possesses designed paint bands having a thickness of 10 microns printed around the grid, thus obtaining a device in which the thickness between the two slides is 10 microns.

3. The disposable device according to Claims 1 or 2, wherein said paint is of an epoxy type.

4. The disposable device according to Claims 1 or 2, wherein said grid is marked by a high resolution laser beam to obtain an exacf 10x10 squares of a total area of 1 square millimeter.

5. The disposable device according to Claims 1 to 4, wherein the grid lines have a width of up to 7 microns.

6. The disposable device according to Claims 1 to 5, wherein said lower surface possesses two grids.

7. The disposable device according to Claim 1 to 6, wherein said lower flat surface possesses a continuous round protrusion at a height of 10 microns around said grid.

8. The disposable device according to Claims 2 to 7, wherein said thickness of 10 microns is obtained by inserting a middle slide with a round hole to surround the grid.

9. The disposable device according to Claims 1 to 8, wherein said solid surfaces are strongly attached by gluing.

10. The disposable device according to Claims 1 to 9, being made from glass.

**11.** The disposable device according to Claims 1 to 10, wherein the device with the sperm cells sample is inserted into a temperature control holding unit which is placed under the microscope lens, enabling the counting and evaluation of the sperm sample at a controlled constant temperature.

**12.** The disposable device according to Claim 11, wherein said temperature controlled holding unit is connected via an electrical cable to a temperature control instrument which regulates the temperature of the holding unit.

**13.** The disposable device according to Claim 12, wherein said temperature of the holding unit is kept in the range of between 35°C-37°C.

**14.** A disposable device for an objective determination of sperm cells quality, substantially as described in the specification, drawings and any one of Claims 1 to 13.

Fig.1

Fig.2

Fig.3

Fig. 4